# EUROPÄISCHE PATENTSCHRIFT

(11) **EP 1 387 165 B1**
(45) Veröffentlichungstag und Bekanntmachung des Hinweises auf die Patenterteilung: **26.12.2012**
(21) Anmeldenummer: 03017035.1
(22) Anmeldetag: 26.07.2003
(51) Int. Cl.: G01N 33/28, F16H 57/04, G01N 27/62

(54) **Verfahren und Vorrichtung zur Überwachung der Qualität von Schmieröl mittels Ionenmobilitätsspektroskopie**
Method and device for monitoring the quality of lubricants using ion mobility spectroscopy
Méthode et appareil pour contrôler la qualité des lubrifiants par un spectromètre de mobilité d'ions

(30) Priorität: 02.08.2002 DE 10235612
(43) Veröffentlichungstag der Anmeldung: 04.02.2004
(73) Patentinhaber: Siemens AG, 80506 München (DE)
(72) Erfinder: Becker, Edwin, Dr., 48734 Reken (DE); Deppermann, Klaus Peter, 44577 Castrop-Rauxel (DE); Dahms, Gerd, 47138 Duisburg (DE); Engel, Lothar, Dr., 53347 Alfter (DE); Zundel, Mark, 47198 Duisburg (DE)
(74) Vertreter: Maier, Daniel Oliver

(56) Entgegenhaltungen:
- WO-A-01/73816
- WO-A-96/18893
- WO-A-98/53296
- US-B1- 6 225 623
- US-B1- 6 421 588
- LEVERMORE D M ET AL: "Headspace analysis of engine oil by gas chromatography/mass spectrometry." ANALYTICAL CHEMISTRY. UNITED STATES 15 MAR 2001, Bd. 73, Nr. 6, 15. März 2001 (2001-03-15), Seiten 1361-1365, XP002253739 ISSN: 0003-2700
- THOMAS FRÜH: "Neuer Schnuppersensor wacht über Ölzustand" RWTH-AACHEN, [Online] Seiten 1-2, XP002253740 AACHEN Gefunden im Internet: <URL:http://www.rwth-aachen.de/zrs/v0001/d ez3_pm2003_schnuppersensor.htm> [gefunden am 2003-09-08] -& T. MEINDORF, F. PLENERT: "A new, quasi-continuous measuring technique to determine the condition of hydraulik fluids" ÖLHYDRAULIK UND PNEUMATIK, [Online] Bd. 47, Nr. 7, 2003, Seiten 1-25, XP002253741 Gefunden im Internet: <URL:http://www.rwth-aachen.de/zrs/v0001/d ez3_pm2003_images_RWTH-sensor.pdf> [gefunden am 2003-09-08]
- ZUNDEL M: "Condition Monitoring at the machine element 'Oil'", VDI BERICHTE, DUESSELDORF, DE, no. 1904 II, 1 January 2005 (2005-01-01), pages 1387-1394, XP009149479, ISSN: 0083-5560

## Beschreibung

Die Erfindung betrifft ein Verfahren und eine Vorrichtung zur Überwachung der Qualität von in einem Getriebe befindlichem, Wirkstoffe enthaltenden Schmieröl.

Die Schmierölqualität ist im Maschinen- und Anlagenbau und insbesondere in der Getriebetechnik eine wesentliche Einflussgröße, die die Verfügbarkeit, die Zuverlässigkeit und die Sicherheit des gesamten Antriebsstranges oder der geschmierten Baukomponente bestimmt. Öllösliche Wirkstoffe, wie z. B. verschiedene Extrem-Pressure- und Anti-Wear-Additive, werden Mineralölen, Mineralölprodukten oder Syntheseölen zugegeben, um die Schmierwirkung oder die chemischen Eigenschaften zu verbessern. Qualitätsunterschiede in den verschiedenen Schmierölen stellen ein Wettbewerbskriterium dar. Die Erfahrung in der Wartung von Getrieben zeigt, dass auch die besten Schmieröle altern und gewechselt werden müssen. Dabei geht man schrittweise von den zeitgeplanten Ölwechselintervallen mehr zu den zustandsabhängigen Ölwechselfristen über. Kriterium ist die klassische Ölanalyse, mit der die physikalischen und chemischen Parameter des Schmieröls analysiert werden.

Eindeutig meßbare Kriterien, die darüber Auskunft geben könnten, wann die Qualität eines Öls unzureichend wird, existieren jedoch noch nicht. Aus dem Grunde ist es z. B. in der Windkrafttechnik Standard und Vorschrift, dass bei Windkraftanlagen die Türme regelmäßig bestiegen, Ölproben gezogen und die Ölqualitäten anschließend im Laboratorium bestimmt werden. Verschlechtern sich einzelne Parameter des Öls, wird schon aus Gründen der Sicherheit das Schmieröl gewechselt. Um den geeigneten Zeitpunkt eines Ölwechsels feststellen zu können, bedarf es bei diesen Analysen schon eines gut ausgerüsteten Analyselaboratoriums sowie einer exakten Probennahme. Erfasst werden die Viskosität, die Säurezahl, die Menge an Fremdpartikeln und deren Zusammensetzung. Diese Eigenschaften können nur mit sehr teuren Analysegeräten ermittelt und nur vom Spezialisten bewertet werden.

Sofortige Angaben über etwaige Gefährdungen des Getriebes aufgrund unzureichender Qualität des alternden Öls sind nur im Endstadium der Gebrauchsdauer des Schmieröls möglich. In der Fachliteratur gibt es eine große Anzahl von Wechselkriterien, die einander zum Teil widersprechen.

Aus der W09618893 ist es bekannt, Substanzen, insbesondere Alkohole, im Dampf eines Fermentors mittels IMS zu analysieren.

W09853296 offenbart eine spezielle Desorptionsmethode mittels Bestrahlung in Verbindung mit Massenspektroskopie, wobei in einer Liste möglicher Anwendungen die Untersuchung der Zusammensetzung von in Form von einzelnen oder mehrfachen Monolagen vorliegenden Schmierstoffen als Funktion des Abriebs (S.19,Z.5-8) und in einer Liste von Massenspektrometern auch lonenmobilitätsspektrometer aufgeführt sind (S.16,Z.24-27)

Der Artikel "Headspace Analysis of Engine Oil by Gas Chromatography/Mass Spectrometry (D.M.Levermore et al. in Analytical Chemistry, Vol.73, No.6, March 15, 2001) und die US6421588 offenbaren beide ein Verfahren zur Überwachung der Qualität von in einer Maschine befindlichem Schmieröl, wobei eine Probe aus dem Schmieröl austretenden Dampfes entnommen wird, die Probe des aus dem Schmieröl austretenden Dampfes einem Gaschromatographen/Massenspektrometer zugeführt wird, die Probe auf in der Dampfphase über dem Schmieröl vorhandene Stoffe analysiert wird und die Veränderung des Gehaltes und der Art der analysierten Stoffe in der Probe gegenüber vorbestimmten Stoffen in der Dampfphase des ungebrauchten Schmieröls als Ist-Zustand für die Alterung des Schmieröls durch Vergleich ausgewertet wird.

Der Erfindung liegt die Aufgabe zugrunde, ein Verfahren und eine Vorrichtung bereit zu stellen, die vor ort eine schnelle und zuverlässige Überwachung der Qualität des in einem Getriebe befindlichen Schmieröls ermöglichen.

Die Aufgabe wird bei einem gattungsgemäßen Verfahren erfindungsgemäß durch die kennzeichnenden Merkmale des Anspruches 1 gelöst. Eine Vorrichtung zur Durchführung des Verfahrens ist Gegenstand des Anspruches 5. Vorteilhafte Ausgestaltungen der Erfindung sind in den Unteransprüchen angegeben.

Der erfindungsgemäßen Lösung liegt die Erkenntnis der Erfinder zugrunde, dass sich die schmieröle je nach Herkunft, Alter und zusammensetzung durch ihren Geruch unterscheiden lassen. Der Geruch kommt unter anderem durch die Wirkstoffe und deren Abbauprodukte zustande, die den Hochleistungsschmierölen als Eigenschaftsverbesserer beigemischt werden. Sinkt der Gehalt an Wirkstoffen im Schmieröl während des Betriebes durch Alterung, so ändert sich auch die Zusammensetzung der Dampfphase über dem Schmieröl. Gemäß der Erfindung wird nun vorgeschlagen, die in der Dampfphase über dem Schmieröl vorhandenen Stoffe mit einem Ionenmobilitätsspektrometer zu analysieren. Die Ionenmobilitätsspektroskopie ist z. B. aus der DE 195 15 270 A an sich bekannt und wird zur Analyse von Spurengasen eingesetzt. Im Rahmen der Erfindung wird die Ionenmobilitätsspektroskopie dazu benutzt, um die in der Dampfphase über dem Öl vorhandenen Stoffe zu analysieren und mit Vergleichswerten zu vergleichen, die bei ungebrauchten Schmierölen gefunden werden. Aus der Änderung des Gehaltes an flüchtigen Komponenten gegenüber dem Ausgangszustand wird auf die geänderte Qualität des Schmieröls geschlossen. Die Art der Bestimmung des Qualitätszustandes des Öls mit Hilfe des Ionenmobilitätsspektrometers ist zuverlässig und schnell, lässt sich vor Ort durchführen und fernsteuern. Die Messergebnisse können an einen beliebigen Ort übertragen werden, so dass eine Fernüberwachung möglich wird.

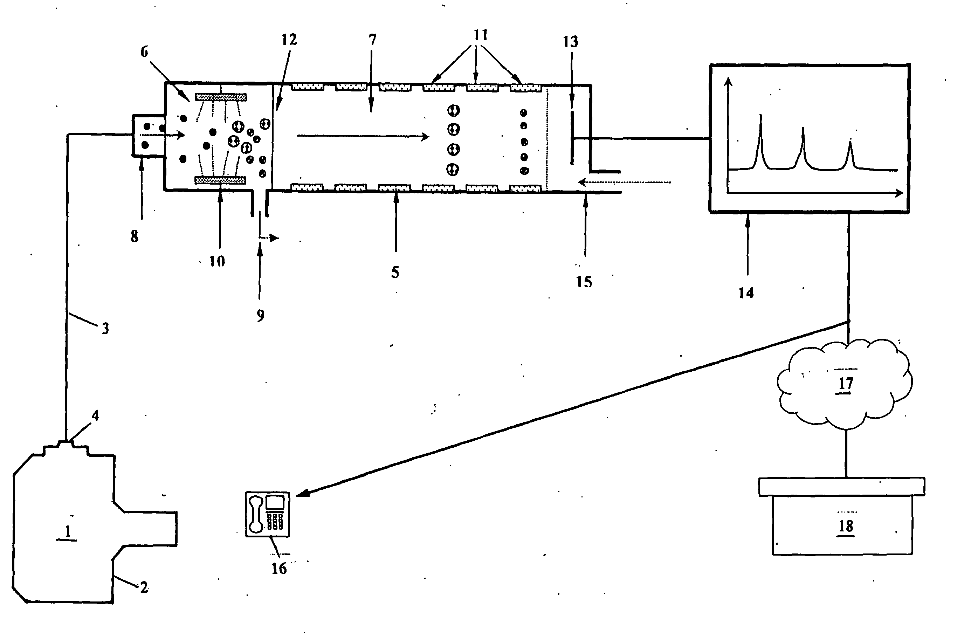

Ein Ausführungsbeispiel der Erfindung ist in der Zeichnung dargestellt und wird im Folgenden näher erläutert.

Die einzige Figur der Zeichnung zeigt das Schema einer Vorrichtung zur Überwachung der Schmierölqualität.

Ein Getriebe 1 oder eine Maschine ist von einem Gehäuse 2 umgeben, das bis zu einem vorgegebenen Ölstand mit Schmieröl zur Schmierung der rotierenden Teile innerhalb des Gehäuses 2 gefüllt ist. Das Getriebe 1 ist vorzugsweise in einer nicht ständig von Personal überwachten Anlage, z. B. in einer Windkraftanlage, aufgestellt.

Das Gehäuse 2 des Getriebes 1 ist mit einer Probeentnahmeleitung 3 versehen, über die eine Probemenge an Schmieröl oder an Öldampf, der sich in dem Gehäuse 2 oberhalb des Ölbades bildet, entnommen wird. Die Probeent-nahmeleitung 3 kann an den Ölentlüfter 4 angeschlossen sein, mit dem ein Druckausgleich für das Getriebe 1 geschaffen wird. Die Probeentnahmeleitung 3 kann auch an einen anderen Stutzen angeschlossen werden, der an dem Gehäuse 2 oberhalb des Ölbades angebracht ist.

Die Probeentnahmeleitung 3 ist zu einem Ionenmobilitätsspektrometer 5 geführt, das aus einem Reaktionsraum 6 und einer Driftkammer 7 besteht. Der Reaktionsraum 6 ist mit einem Probeneinlass 8 und einem Auslass 9 versehen und nimmt eine Ionisationsquelle 10 auf. Die Driftkammer 7 ist auf der Innenseite mit Driftringen 11 versehen, die paarweise geschaltet und mit einer Hochspannungsgleichstromquelleverbunden sind. Dadurch wird in der Driftkammer 7 ein axiales elektrostatisches Feld aufgebaut.

Der Reaktionsraum 6 ist von der Driftkammer 7 durch ein Schaltgitter 12 getrennt, das im Prinzip eine Vielzahl von elektrisch leitenden Stegen aufweist, die paarweise geschaltet und mit einer Spannungsquelle verbunden sind. Die Stege sind durch Durchbrüche voneinander getrennt. An dem dem Schaltgitter 12 gegenüberliegenden Ende ist in der Driftkammer 7 ein Ionendetektor 13 angeordnet. Der Ionendetektor 13 ist über einen Verstärker mit einer Auswerteeinheit 14 verbunden. Die Auswerteeinheit 14 kann mit einer Fernüberwachung verbunden sein.

In dem Reaktionsraum 6 werden die in dem eintretenden Probestrom enthaltenen Moleküle mit Hilfe der Ionisationsquelle 10 ionisiert. Das Schaltgitter 12 wird durch Anlegen eines bestimmten Spannungsmusters abwechselnd für die Ionen durchlässig oder gesperrt geschaltet. In der Durchlassphase treten die Ionen in die Driftkammer 7 ein, werden dort getrennt und wandern gegen ein durch den Driftgaseinlass 15 zugeführtes Driftgas, beispielsweise Luft, Stickstoff oder dergleichen, in Richtung auf den Ionendetektor 13. Die auf den Ionendetektor 13 auftreffenden Ionen verursachen dort einen Signalstrom, der in der Auswerteeinheit 14 abgespeichert und ausgewertet wird. Je nach dem Gehalt oder der Art der zu untersuchenden Stoffe ergeben sich unterschiedliche Spektren in der Auswerteeinheit 14.

Die in dem Öldampf enthaltenen Stoffe werden nach dem oben beschriebenen Verfahrensprinzip analysiert, wobei sich je nach dem Gehalt und der Art der Stoffe ein bestimmtes Spektrum ergibt, das in der Auswerteeinheit 14 angezeigt wird. Da einem Schmieröl verschiedene Wirkstoffe zugesetzt sind, stellen die im Öldampf enthaltenen und sich im analysierten Spektrum deutlich zeigenden Abbauprodukte ein wesentliches Indiz für den Zustand des Schmieröls dar. Verändert sich im Vergleich mit dem Ausgangszustand (Soll-Zustand) des ungebrauchten Schmieröls das Aussehen der Spektren (Ist-Zustand), kann anhand der zugehörigen Spektren erfaßt werden, wie die betriebswichtige Additivierung des Schmieröls abgebaut wird und das Schmieröl gealtert ist oder sogar Wasser enthält.

Die durch das Ionenmobilitätsspekktrometer 5 gewonnenen Messergebnisse bezüglich der Änderung in dem Gehalt und der Art der im Öldampf enthaltenen Stoffe werden in der Auswerteeinheit 14 als Ist-Zustand für die Alterung des Schmieröls gegenüber ungebrauchtem Schmieröl ausgewertet. Beim Erreichen oder Überschreiten vorgegebener Grenzwerte in der Auswerteeinheit wird ein Warnsignal hervorgerufen, beispielsweise für das Wartungspersonal. Die ausgewerteten Messergebnisse können auch dem Wartungspersonal mittels bekannter Telediagnosetechnik übermittelt werden.

Als Ergebnis der Auswertung kann in einer Messwarte 16 ein Alarm ausgelöst werden. Die Messergebnisse können auch an eine Datenfernübertragung 17 oder das Internet weitergeleitet und in einer Fernüberwachungsstation 18 abgerufen werden. Ergibt sich aus den ausgewerteten und übermittelten Messergebnissen ein kritischer Zustand hinsichtlich der Qualität des Schmieröls, so besteht Handlungsbedarf, was dem Wartungspersonal durch ein Warnsignal angezeigt wird. Dann wird im Bedarfsfall dem gealterten Schmieröl frischer Wirkstoff zugesetzt, oder es wird das Schmieröl ausgewechselt.

## Patentansprüche

1. Verfahren zur Überwachung der Qualität von in einem Getriebe (1) befindlichem, Wirkstoffe enthaltendem Schmieröl, wobei aus dem Getriebe (1) eine Probe aus dem Schmieröl austretenden Dampfes entnommen wird, die Probe des aus dem Schmieröl austretenden Dampfes einem Ionenmobilitätsspektrometer (5) zugeführt wird, die Probe auf in der Dampfphase über dem Schmieröl vorhandene Stoffe analysiert wird und die Veränderung des Gehaltes und der Art der analysierten Stoffe in der Probe gegenüber vorbestimmten Stoffen in der Dampfphase des ungebrauchten Schmieröls als Ist-Zustand für die Alterung des Schmieröls durch Vergleich ausgewertet wird.

2. Verfahren nach Anspruch 1,
**dadurch gekennzeichnet, dass** das Schmieröl nach der Auswertung der erfassten Messergebnisse durch Vergleich mit vorgegebenen Grenzwerten klassifiziert wird.

3. Verfahren nach Anspruch 1,
**dadurch gekennzeichnet, dass** dem Schmieröl nach der Auswertung der erfassten Messergebnisse durch Vergleich mit vorgegebenen Grenzwerten Wirkstoffe zugesetzt werden.

4. Verfahren nach Anspruch 1,
**dadurch gekennzeichnet, dass** das Schmieröl nach der Auswertung der erfassten Messergebnisse durch Vergleich mit vorgegebenen Grenzwerten gewechselt wird.

5. Vorrichtung zur Durchführung des Verfahrens nach Anspruch 1, umfassend ein Getriebe und ein Ionenmobilitätsspektrometer, wobei eine Probeentnahmeleitung (3) an den Innenraum des Getriebes (1) oberhallo des diespiegels angeschlossen ist, die Probeentnahmeleitung (3) mit einem Ionenmobilitätsspektrometer (5) verbunden ist und an das Ionenmobilitätsspektrometer (5) eine Auswerteeinheit (14) angeschlossen ist, die eine Auswertung gemäß Anspruch 1 durch führt.

6. Vorrichtung nach Anspruch 5,
**dadurch gekennzeichnet, dass** die Auswerteeinheit (14) mit einer Messwarte (16) verbunden ist.

7. Vorrichtung nach Anspruch 5,
**dadurch gekennzeichnet, dass** die Auswerteeinheit (14) mit einer Fernüberwachungsstation (18) verbunden ist.

8. Vorrichtung nach Anspruch 7,
**dadurch gekennzeichnet, dass** bei dem Getriebe (1) mit einen Ölentlüfter (4) die Probeentnahmeleitung (3) an den Ölentlüfter (4) angeschlossen ist.

## Claims

1. Method for monitoring the quality of lubricating oil, which is located in a transmission (1) and contains active ingredients, wherein a sample of the vapour emerging from the lubricating oil is taken from the transmission (1), the sample of the vapour emerging from the lubricating oil is fed to an ion mobility spectrometer (5), the sample is analysed with respect to substances present in the vapour phase over the lubricating oil and the change in terms of content and type of the analysed substances in the sample is evaluated by comparison with respect to predetermined substances in the vapour phase of the unused lubricating oil as actual state for the ageing of the lubricating oil.

2. Method according to Claim 1, **characterized in that** the lubricating oil is classified after the evaluation of the captured measurement results by comparison with prespecified limit values.

3. Method according to Claim 1, **characterized in that** active ingredients are added to the lubricating oil after the evaluation of the captured measurement results by comparison with prespecified limit values.

4. Method according to Claim 1, **characterized in that** the lubricating oil is changed after the evaluation of the captured measurement results by comparison with prespecified limit values.

5. Apparatus for carrying out the method according to Claim 1, comprising a transmission and an ion mobility spectrometer, wherein a sampling line (8) is connected to the interior of the transmission (1) above the oil level, the sampling line (3) is connected to an ion mobility spectrometer (5) and an evaluation unit (14), which performs an evaluation according to Claim 1, is connected to the ion mobility spectrometer (5).

6. Apparatus according to Claim 5, **characterized in that** the evaluation unit (14) is connected to a measurement station (16).

7. Apparatus according to Claim 5, **characterized in that** the evaluation unit (14) is connected to a remote monitoring station (18).

8. Apparatus according to Claim 7, **characterized in that** in the case of the transmission (1) with an oil bleeder (4), the sampling line (3) is connected to the oil bleeder (4).

## Revendications

1. Procédé de contrôle de la qualité de l'huile lubrifiante se trouvant dans une transmission ( 1 ) et contenant des substances actives, dans lequel on prélève de la transmission ( 1 ) un échantillon de vapeur sortant du lubrifiant, on envoie l'échantillon de la vapeur sortant du lubrifiant à un spectromètre ( 5 ) de mobilité d'ions, on analyse l'échantillon en ce qui concerne des substances présentes dans la phase vapeur au dessus du lubrifiant et on évalue par comparaison, comme étant l'état réel du vieillissement du lubrifiant, la modification de la teneur et du type des substances analysées dans l'échantillon par rapport à des substances déterminées à l'avance dans la phase vapeur du lubrifiant inutilisé.

2. Procédé suivant la revendication 1,
**caractérisé en ce que** l'on classe le lubrifiant en fonction de l'évaluation des résultats de la mesure détectés par comparaison avec des valeurs limites prescrites.

3. Procédé suivant la revendication 1,
**caractérisé en ce que** l'on ajoute des substances actives au lubrifiant après évaluation des résultats de la mesure détectés par comparaison avec des valeurs limites prescrites.

4. Procédé suivant la revendication 1,
**caractérisé en ce que** l'on remplace le lubrifiant après évaluation des résultats de la mesure détectés par comparaison avec des valeurs limites prescrites.

5. Dispositif pour effectuer le procédé suivant la revendication 1, comprenant une transmission et un spectromètre d'immobilité d'ions, dans lequel un conduit ( 3 ) de prélèvement d'échantillon est raccordé à l'espace intérieur de la transmission ( 1 ) au dessus du niveau du lubrifiant, le conduit ( 3 ) de prélèvement d'échantillon communique avec un spectromètre ( 5 ) d'immobilité d'ions et le spectromètre ( 5 ) d'immobilité d'ions est raccordé à une unité ( 14 ) d'évaluation qui effectue une évaluation suivant la revendication 1.

6. Procédé suivant la revendication 5,
**caractérisé en ce que** l'unité ( 14 ) d'évaluation est reliée à un poste ( 16 ) de contrôle de mesure.

7. Procédé suivant la revendication 5,
**caractérisé en ce que** l'unité ( 14 ) d'évaluation est reliée à un poste ( 18 ) de télécontrôle.

8. Procédé suivant la revendication 7,
**caractérisé en ce que**, pour la transmission ( 1 ) ayant un purgeur ( 4 ) de lubrifiant, le conduit ( 3 ) de prélèvement d'échantillon est raccordé au purgeur ( 4 ) de lubrifiant.
